# EUROPEAN PATENT APPLICATION

(11) **EP 0 988 843 A1**
(43) Date of publication of application: **29.03.2000**
(21) Application number: 99306590.3
(22) Date of filing: 20.08.1999
(51) Int. Cl.: A61F 5/448

(54) **Ostomy coupling**

(30) Priority: 28.08.1998 GB 9818893
(71) Applicant: BRISTOL-MYERS SQUIBB COMPANY, Princeton, NJ 08543-4000 (US)
(72) Inventor: Steer, Peter Leslie, East Grinstead, Surrey RH10 4JZ (GB); Hollands, Keith G. M., Sompting, Sussex (GB)
(74) Representative: Holmes, Miles Keeton

(57) **Abstract**

An ostomy coupling (10) is described in which a body-side coupling member (12) has walls (20, 22) defining a channel (24) for receiving the projecting annular formation (48) of the bag-side coupling member (14). A locating rib (26) projects from the floor of the channel, for co-operating with a groove (58) of the bag-side member to centre and prevent tilting of the coupling members. The bag-side member has a deformable wiper seal (56) which seals against the inner wall (20) of the body-side member, and wipes clean the wall (20) during assembly and separation of the coupling members. The outer wall (22) of the bag-side coupling member might be deformable, and a finger tab (82) might be provided to facilitate separation of the coupling members if desired. The bag-side coupling member (14) has an integral vent filter housing (60).

## Description

This invention relates to an ostomy coupling. Such couplings are used to releasably couple an ostomy pouch to a pad worn on the peristomal region of the ostomate. The term "ostomy" includes at least colostomy, urostomy and ileostomy.

In one form, the invention relates to a so-called two piece coupling consisting substantially of only two coupling members, one for the bag-side of the coupling and the other for the body-side of the coupling.

In one aspect, the invention provides an ostomy coupling comprising: a first coupling member for use as the body-side coupling member and a second coupling member for use as the bag-side coupling member; the first coupling member having a stomal aperture therein, first and second walls outside the aperture defining a channel therebetween, and a locating rib positioned between the first and second walls, the first wall comprising a plurality of spaced apart undercut projections on the wall face facing the second wall; and the second coupling member comprising a stomal aperture and a formation which, when the coupling members are assembled, is received in the channel between the first and second walls of the first coupling member, the formation including a locking rib for fitting between the first wall and the locating rib of the first coupling member, the locking rib including a locking formation for forming a mechanical interlock with the projections of the first wall, and the formation further comprising a deformable wiper for forming a seal against the second wall of the first coupling member, the deformable wiper and the locking rib being spaced apart by a groove in which, in use when the coupling members are assembled, the locating rib of the first coupling member is received to guide the coupling members relative to each other.

The above design can provide a number of significant advantages which have not been achieved hitherto in combination:
Firstly, the locating rib and the groove can aid alignment of the coupling members relative to each other, and can prevent one coupling member from tipping relative to the other. A problem which has been encountered previously is that, as the user presses the coupling members into engagement together, one member tips or cants slightly. This means that locking engagement and the seal effect would not be achieved all around the coupling. The coupling might then be prone to accidental separation, and unpleasant smells could leak through the uncompleted or unreliable seal.
Secondly, the seal wiper being on the body-side coupling member is able to wipe, and hence clean, the respective surface of the bag-side member. This is particularly important because the body-side member will tend to be left in position and changed less frequently than the bag-side member. For example, the body-side member might be retained for up to a week, whereas the ostomy bag (and hence the bag-side member) might be renewed once or twice daily (or even more frequently). The cleaning action of the wiper seal can prevent the unhygenic and unpleasant build-up of fecal matter which might contact, and accumulate on, the body-side member.

Preferably, at least a portion of the first wall of the first coupling member is resiliently deformable, to facilitate release of the engagement between the projections and the locking step when the coupling members are to be separated. The term "deformable" is intended to mean that the first wall (or portion thereof) is more deformable than the second wall. Such a deformable characteristic enables the channel to "open" or become wider to facilitate the release. This can provide similar behaviour to the natural opening of the channel when the channel is used on the bag-side of the coupling as in some conventional arrangements. When a deformable wall (or wall portion) is used as described above, the engagement between the locating rib and the groove is especially important in centering one coupling member relative to the other, because the deformable nature of the outer wall could otherwise allow lateral movement or mis-positioning of one coupling member relative to the other. Such movement or mis-positioning might reduce the effectiveness of the seal which might rely on close fitting tolerances.

Additionally, or alternatively, it is preferred that the first wall (or a portion thereof) be thinner than the second wall (or a portion thereof). If desired, such a difference in wall thickness may be used to make the first wall more deformable than the second wall.

Preferably, at least one of the first and second walls is tapered or has a tapered face.

For example, the face of the second wall engaged by the seal wiper may taper outwardly towards the mouth of the channel defined by the first and second walls. Such a taper can enable the coupling member to be more easily released from the mould after moulding. The taper can also provide a progressive increase in the sealing force applied on, or by, the seal wiper as the coupling members are pressed fully into the engagement with each other. The engagement between the locating rib and the groove is then especially important to ensure that the increased sealing force does not interfere with the interlocking action and the release action between the locking rib or the bagside coupling member, and the locking projection of the bodyside coupling member.

Preferably, the locating rib is positioned closer to the first wall than to the second wall. This can provide more room to accommodate the seal wiper of the bagside coupling members when the two members are assembled.

In some embodiments, one or more finger tabs may be provided on the first wall to enable a user to "open" the channel between the first and second walls by pressing on the tabs(s). This can further facilitate separation of the coupling members.

In a closely related aspect, the invention provides an ostomy coupling comprising: a first coupling member for use as the body-side coupling member and a second coupling member for use as the bag-side coupling member; the first coupling member having a stomal aperture therein, first and second walls outside the aperture defining a channel therebetween, and a locating rib positioned between the first and second walls, the first wall comprising a locking projection on the wall face facing the second wall; and the second coupling member comprising a stomal aperture and a formation which, when the coupling members are assembled, is received in the channel between the first and second walls of the first coupling member, the formation including a locking rib for fitting between the first wall and the locating rib of the first coupling member, the locking rib including a locking formation for forming a mechanical interlock with the projection of the first wall, and the formation further comprising a deformable wiper for forming a seal against the second wall of the first coupling member, the deformable wiper and the locking rib being spaced apart by a groove in which, in use when the coupling members are assembled, the locating rib of the first coupling member is received to guide the coupling members relative to each other; wherein at least a portion of the first wall is resiliently deformable to facilitate engagement and disengagement of the coupling members.

In a closely related third aspect, the invention provides an ostomy coupling comprising: a first coupling member for use as the body-side coupling member and a second coupling member for use as the bag-side coupling member; the first coupling member having a stomal aperture therein, first and second walls outside the aperture defining a channel therebetween, and a locating rib positioned between the first and second walls, the first wall comprising a projection on the wall face facing the second wall and a finger tab projecting laterally outwardly from the first wall; and the second coupling member comprising a stomal aperture and a formation which, when the coupling members are assembled, is received in the channel between the first and second walls of the first coupling member, the formation including a locking rib for fitting between the first wall and the locating rib of the first coupling member, the locking rib including a locking formation for forming a mechanical interlock with the projection of the first wall, and the formation further comprising a deformable wiper for forming a seal against the second wall of the first coupling member, the deformable wiper and the locking rib being spaced apart by a groove in which, in use when the coupling members are assembled, the locating rib of the first coupling member is received to guide the coupling members relative to each other.

Preferably, the finger tab is located adjacent to, or towards, the distal edge of the first wall.

In preferred embodiments, the seal between the two coupling members is achieved independently of the mechanical interlock between the members. A mechanical interlock is only formed at one side of the channel; the other side ofthe channel is used to form the seal. This can separate the sealing and interlocking functions of the coupling members, and thereby improve the reliability of the coupling in use, and also make the coupling easier to use (during assembly and disassembly of the members).

Many designs of ostomy coupling are known in the art, reference being made to GB-A-2254785, GB-A-2296662, GB-A-2151482 and WO-A-93/23229. However, none of the known prior art designs can provide all of the advantages described above. For example, the first mentioned document describes a two-piece channel in which a channel with a locating rib is provided on the bag-side coupling member for receiving a projecting rib with an alignment groove of the body-side member. However, this arrangement relies on the "peeling" open effect to release the rib from the channel when the bag-side member is peeled away. Moreover, the profile of the channel is very difficult to mould, and the arrangement does not achieve automatic cleaning of the body-side coupling member when a bag is fitted or removed.

Embodiments of the invention are now described by way of example only, in which:
Fig. 1 is a partial schematic section through a first embodiment of coupling with the members in a separated condition;
Fig. 2 is a partial schematic section illustrating full engagement of the members of Fig. 1;
Fig. 3 is a plan view of the bag-side member;
Fig. 4 is a plan view of the body-side member;
Fig. 5 is a schematic section showing the filter housing;
Figs. 6a-d are partial schematic sections through a modified embodiment showing assembly of the coupling members;
Fig. 7 is a partial schematic section showing a third embodiment of coupling with the coupling members in a separated condition;
Fig. 8 is a partial schematic section showing full engagement of the coupling members of Fig. 7; and
Fig. 9 is a schematic section similar to Fig. 8, but showing deflection of the channel outer wall.

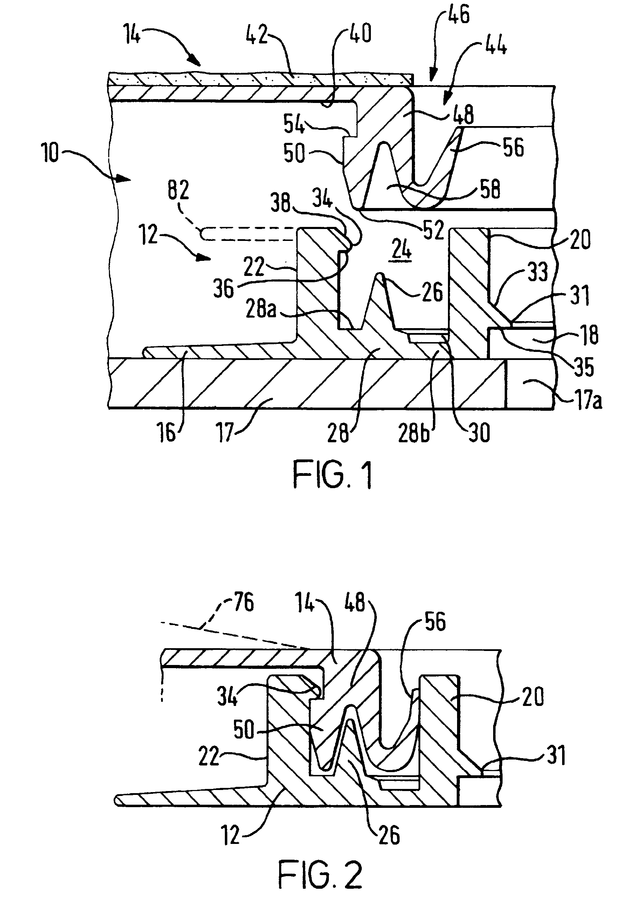

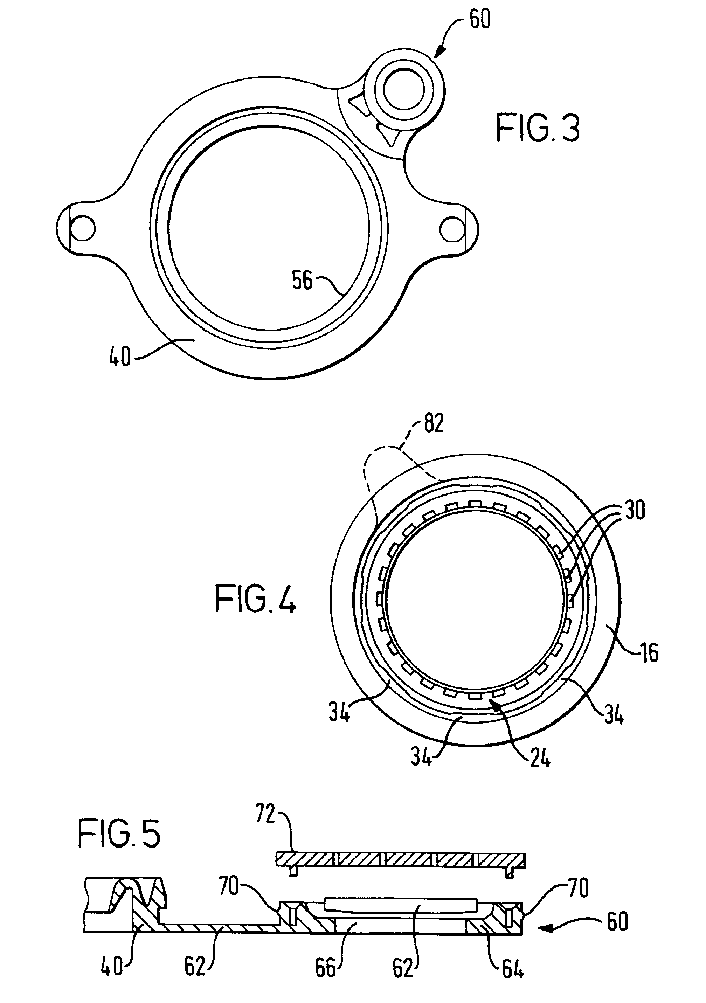

Referring to Figs. 1 to 5, an ostomy coupling 10 consists generally of a first body-side coupling member 12 and a second bag-side coupling member 14.

The body-side member 12 comprises an annular flange 16 which is attached to an adhesive "wafer" or pad 17 for attachment to a person's skin. The pad 16 may be made of Stomadhesive or other known skin adhesives; such adhesives are well known to the skilled man and so are not described further here.

The flange 16 includes a stomal aperture 18 bounded by a generally cylindrical inner (second) wall 20. Positioned concentrically outside the inner wall is an outer (first) wall 22, defining a generally annular channel 24 between the walls. A locating rib 26 projects from the floor 28 of the channel 24, to a lesser extent than the inner and outer walls 20 and 22. In this embodiment, the locating rib 26 has a generally tapered shape, but it will be appreciated from later description that other shapes of rib 26 may be used as desired.

The floor 28 of the channel includes a first radially outer portion 28a between the rib 26 and the outer wall 22, and a second radially inner portion 28b between the rib 26 and the inner wall 20. The radially inner portion includes a plurality of, in this embodiment regularly spaced, recesses 30 for facilitating moulding of the coupling member as described in WO-A-93/23229 and also described briefly below.

The inner wall 20 carries, on its inner face 32, a plurality of spaced apart projections 34. These serve to form an interlock with a corresponding formation of the bag-side member. Each projection 34 includes a generally flat or undercut rear surface 36, and a upper ramp surface 38.

As described in WO-A-93/23229, during production of the coupling member, the spacing between the projections 34 permits the portions of the moulding tool (not shown) under the projections 34 to be removed from inside the channel 24 by rotating the tool relative to the member (or vice-versa) until the moulding tool portions can be withdrawn through the spaces. The recesses 30 permit keying engagement with the moulded member to achieve the controlled rotation.

An annular lip 31 projects inwardly from the inner surface of the inner wall 20. The purpose of the lip 31 is to provide a mounting attachment for a stoma pressure member (not shown). Such members are known, and are use to press against the stoma. For example, the pressure member might be convex shaped, to press downwardly (i.e. through the opening in the adhesive pad 17). The pressure member can be inserted by being advanced downwardly through the aperture 18 of the coupling member. The lip 31 has an inclined upper surface 33 to guide the edge of the pressure member over the lip 31, until the member snaps into position under the abutment surface 35 of the lip 31. The pressure member is thus retained by the lip 31 and by a projecting portion 17a of the adhesive pad 17.

The bag-side coupling member 14 consists generally of a flange 40 which is attached to a pouch wall 42 by gluing, or welding, or any other suitable fixing. The flange 40 has a stomal aperture 44 which is aligned with a corresponding aperture 46 in the pouch wall 42. Projecting from the flange 40 is a generally annular formation 48 configured to fit within the channel 24 of the bag-side member 12.

The formation includes a radially outer locking rib 50 for fitting between the outer wall 22 and the locating rib 26 of the body-side coupling member. The locking rib 50 includes a generally rounded or tapered tip 52, and a stepped or undercut locking formation 54 for co-operating with the projections 34. The formation also includes a radially inwardly directed seal wiper 56 for forming a seal against the radially outer face of the inner wall 20. The wiper 56 is at least partly deformable or deflectable, and is bent over at its upper end and tapers towards its tip. A generally annular groove 58 between the wiper 56 and the groove 58 is configured to co-operate with the locating rib 26 as described further below.

The bag-side coupling member 14 also includes an integral filter housing 60 for containing a deodorising filter 62. In this embodiment, the filter housing 60 is formed as a satellite to the main flange 40, and is joined to the main flange by one or more connecting webs 62. However, in other embodiments, the housing 60 may be formed as an integral extension of the flange, or be part of the flange.

The housing 60 consists of a floor 64 having an opening 66 in register with a corresponding gas vent 68 in the pouch wall. A peripheral wall 70 projects from the floor, and a cap 72 is securable to the wall 70 to close the housing 60. In this embodiment, the cap is a secured by a snap fit connection, but in other embodiments, other forms of fixing may be used as desired. The cap 72 may be intended to be removable to access or replace the filter 62, or it may be permanently fixed in position.

The use of an integral filter housing 60 can provide advantages in the manufacture of the pouches, in that the filter housing can be secured to the pouch in the same welding step as the coupling member. In many conventional designs, additional manufacturing steps are required to accurately position and weld a filter housing as steps separate from the steps of welding of the coupling member. Such additional steps add to the production cost.

To assemble the coupling members, the bag-side member 14 is positioned against the body-side member 12, and is pressed against the body-side member. Under quite mild pressure, the locking rib 50 is able to pass over the ramp surfaces of the 38 of the projections 34, until the projections 34 snap into engagement against the locking formation 54 of the locking rib 50. However, during such assembly, the engagement will not normally occur simultaneously all around the coupling circumference. Rather, engagement will tend to begin in one angular region, and then progress around the coupling until a complete circle is achieved. Such engagement can result in the bag-side member tilting relative to the axis of the body-side member (as depicted by the broken line 76). In the present embodiment, the locating rib 26 and the groove 58 act to prevent such tilting, and also ensure that the bag-side member 14 is accurately centred on the body-side member 12. Accurate centering is important to enable the seal wiper 56 to engage properly against the surface of the inner wall 20 to form a reliable seal all around the wall. It will be appreciated that the relative shapes of the locating rib 26 and the groove 58 can be varied as desired, but the illustrated smoothly tapered shape is currently preferred for ease of moulding and for optimum guidance during assembly of the coupling members.

To separate the engaged coupling members, the bag-side member 14 is peeled away from the body-side member 12. The plastics material of the body-side and/or bag-side members is able to deform sufficiently to allow release with a modest separation force being applied. However, in normal use, the coupling members are held together securely and will not tend to fall apart accidentally.

During assembly and separation of the coupling members, the wiper 56 on the bag-side wipes the surface of the inner wall 20 of the body-side member 12, thereby cleaning the surface. This is extremely advantageous in keeping the surface clean, and removing any fecal matter which might accumulate on the surface of the wall 20. Such fecal matter would be extremely unhygenic, and would make the coupling very unpleasant to handle when attaching or removing a subsequent pouch.

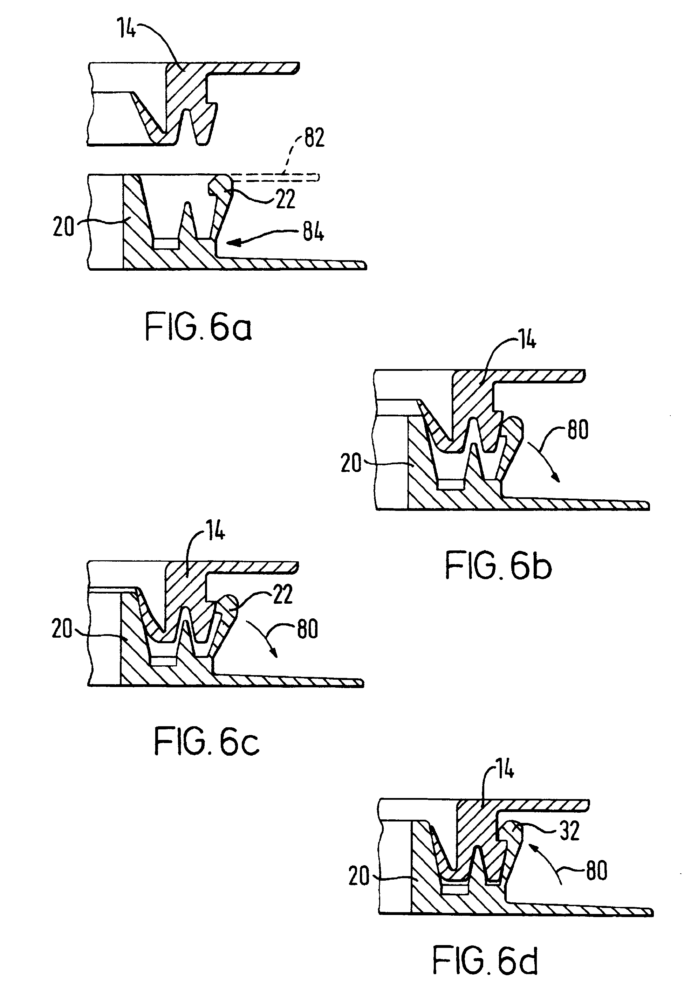

Fig. 6 illustrates a modified design of the body-side member 12. In contrast to the first embodiment in which the inner and outer walls 20 and 22 are generally rigid (or of generally equal rigidity), in the second embodiment, the outer wall 22 is outwardly deformable (illustrated by the arrow 80) to facilitate easier assembly and separation of the coupling members. Such deformation "opens" the channel, thus making it wider during the assembly and separation.

This can provide two effects:
(i) It can reduce the insertion force necessary to assemble together the bagside and bodyside coupling members. In particular, the outer wall deflects when contacted by the tip 52 of the locking rib 50, and thereby reduces the resistance to insertion of the annular formation 48 in the annular channel 24;
(ii) During peeling of the bagside coupling member 14 from the bodyside coupling member 12, it enables the channel 24 to open to release the locking engagement between the coupling members. This can provide an effect similar to that observed when a channel is employed on the bag-side as in some conventional designs; in such designs, the action of peeling away the bag tends inherently to open the channel to start the release of the members. It will be appreciated that such inherent opening of the channel is more difficult to engineer when the channel is implemented on the bodyside coupling member.

In the second embodiment, the locating rib 26 is even more important in centering one coupling member relative to the other. It will be appreciated that the deformable nature of the outer wall 22 means that incorrect centering of the members might otherwise be possible, leading possibly to an ineffective seal.

In the fully engaged position (Fig. 6(D)), the outer wall 22 remains slightly stressed or flexed (i.e. it does not return fully to its natural position as shown in Fig. 6(A)). This maintains a locking force on the bagside member 14 to ensure that the projections 34 of the bodyside member 12 and the locking formation 54 of the bagside member 14 remain in tight engagement, and helps reduce the chances of accidental separation of the coupling members in use.

The outer wall may be made deformable by thinning the wall, for example, particularly at a lower region 84. Alternatively, or additionally, the outer wall may be made from a different plastics material which is less rigid than the material used for the remainder of the coupling member. In this latter case, a multi-shot (for example, two-shot) moulding process may be employed to produce an integral coupling member of the two materials.

In the illustrated embodiment, the outer wall 22 is generally thinner than the inner wall 20 to give it a more deformable characteristic, and the outer wall 22 tapers (thins) towards its lower portion to provide a natural hinging effect.

In contrast to the first embodiment in the second embodiment the face 20a of the inner wall 20 against which the seal wiper 56 bears tapers inwardly towards the base of the channel (i.e. tapers outwardly towards the mouth of the channel). This provides three effects:
(i) It can ease removal of the coupling member from the mould during manufacture;
(ii) It provides a wider mouth for the channel 24, hence making it easier to locate the bagside and bodyside members in register with each other before the coupling members are pressed into locking engagement. This is even more significant bearing in mind that, in the natural position of the outer wall 22 (Fig. 6(a)), the wall 22 is slightly inboard of its fully engaged, locking position (Fig. 6(D)), thus making the channel narrower to some extent before the coupling members are assembled to each other; and
(iii) As best seen in Figs. 6b to 6d, the amount of deflection of the seal wiper 56 (and therefore the magnitude of the sealing force) increases progressively as the bagside coupling member is pressed further on to the bodyside coupling member. The locating rib 26 plays an important role in enabling the increased sealing force to be accommodated without interfering with the interlocking action of the locking rib 50 of the bagside coupling element and the locking projections 34 of the bodyside coupling element. The locating rib 26 can also ensure the coupling members are centred properly.

As indicated by the broken line 82, the body-side member 12 of either of the above embodiments might also include one or more finger tabs for enabling manual opening or "widening" of the channel 24 to facilitate separation of the coupling members. The finger tab 82 is located at, or towards, the distal edge of the outer wall 22, and projects laterally away from the wall 22. Pressing on the tab 82 tends to deform the outer wall 22 to encourage release of the bag-side coupling member 14. Although only one finger tab 82 is illustrated, it will be appreciated that a plurality (for example, four) of such tabs may be used, as desired. It will also be appreciated that the tab 82 does not have to be positioned adjacent to the upper edge of the wall 22. If desired, the tab 82 could be moved away from the upper edge, and somewhat towards the flange 16.

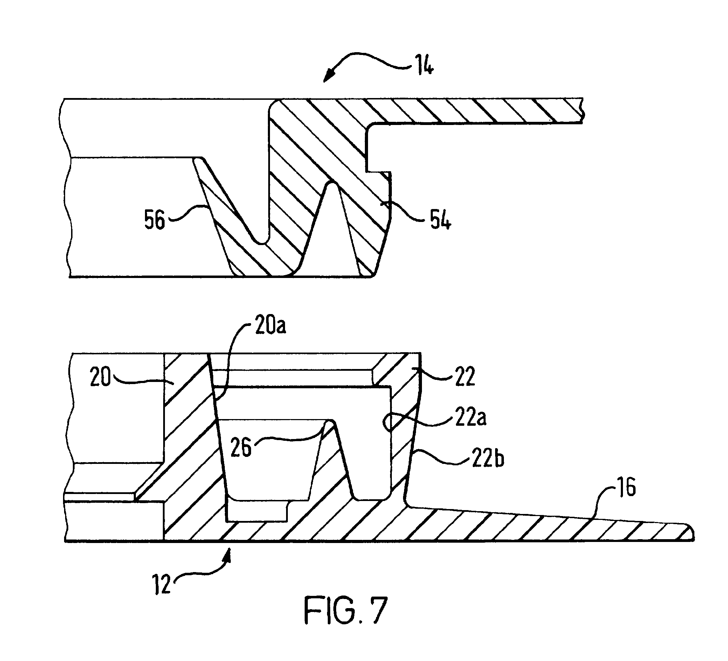

Figs. 7, 8 and 9 show a third embodiment similar to that illustrated in Fig. 6, but with a modified form of deflectable outer wall 22. In contrast to the second embodiment (Fig. 6) in which the outer wall 22 is inclined, in the third embodiment, the inner face 22a of the outer wall 22 is generally upright (i.e. generally parallel with the axis of the coupling). The outer face 22b tapers inwardly towards the flange 16 to provide a region of thinning. This embodiment also includes the inclined sealing face 20a of the inner wall 20 described in the second embodiment.

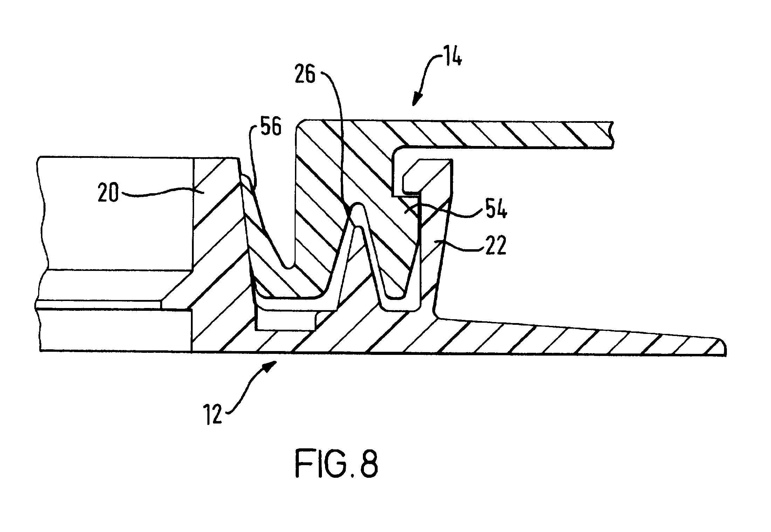

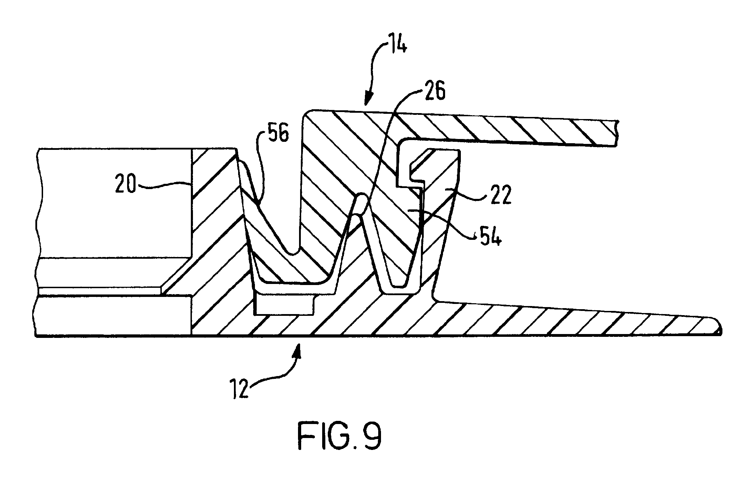

The normal engaged position of the coupling members is shown in Fig. 8. However, if there is any tendency for the outer wall 22 to deflect significantly under the force of the sealing element 56 engaging the (inclined) inner face 20a (as illustrated in Fig. 9), then in a similar manner as that described for the second embodiment, the central rib 26 can serve to locate the bagside coupling member 14 relative to the bodyside member 12 to ensure that the coupling members are accurately centred with respect to each other.

The coupling members of the preferred embodiments are each preferably integrally moulded. Suitable plastics material include, for example, low and high density polyethylene, thermoplastic elastomers (TPE), polyvinyl chloride (PVC), acetyl plastics, ABS, polyamides, and combinations of any of these. For example, different plastics might be used for the two coupling members to give one a generally hard or rigid characteristic, and the other a more pliant characteristic.

The materials used for the coupling members might also depend on the natures of the materials to which they are secured, either by adhesive, or by welding. In a preferred form, the bodyside coupling member is of low density polyethylene (LDPE), and the bagside coupling member is of ethylene vinyl acetate (EVA).

Although the coupling members of the preferred embodiment are generally circular, it will be appreciated that other closed-loop shapes may be used instead if desired.

It will be appreciated that the foregoing description is merely illustrative of preferred forms of the invention, and that many modifications may be made within the scope and/or principles of the invention. Features believed to be of particular importance are defined in the appended claims. However, the Applicant claims protection for any novel feature or idea described herein and/or illustrated in the drawings whether or not emphasis has been placed thereon.

## Claims

1. An ostomy coupling comprising: a first coupling member for use as the body-side coupling member and a second coupling member for use as the bag-side coupling member; the first coupling member having a stomal aperture therein, first and second walls outside the aperture defining a channel therebetween, and a locating rib positioned between the first and second walls, the first wall comprising a locking projection on the wall face facing the second wall; and the second coupling member comprising a stomal aperture and a formation which, when the coupling members are assembled, is received in the channel between the first and second walls of the first coupling member, the formation including a locking rib for fitting between the first wall and the locating rib of the first coupling member, the locking rib including a locking formation for forming a mechanical interlock with the projection of the first wall, and the formation further comprising a deformable wiper for forming a seal against the second wall of the first coupling member, the deformable wiper and the locking rib being spaced apart by a groove in which, in use when the coupling members are assembled, the locating rib of the first coupling member is received to guide the coupling members relative to each other.

2. A coupling according to claim 1, wherein the first wall carries a plurality of spaced apart locking projections.

3. A coupling according to claim 2, wherein the locking projections are generally equally angularly spaced from each other.

4. A coupling according to claim 1, 2 or 3, wherein the or each locking projection has a tapered or curved ramp surface, and a flat or undercut locking surface.

5. A coupling according to any preceding claim, wherein the second wall of the first coupling member is at least partly deformable to facilitate assembly and separation of the coupling members.

6. A coupling according to claim 5, wherein the second wall is outwardly deformable resiliently.

7. A coupling according to any preceding claim, further comprising at least one finger tab on the second wall of the first coupling member, for enabling the application of finger pressure to the second wall.

8. A coupling according to any preceding claim, further comprising a filter housing at least a portion of which is integral with the second coupling member.

9. A coupling according to claim 8, wherein the filter housing is spaced from, but integrally coupled to, the second coupling member.

10. A coupling according to claim 8, wherein the filter housing is formed integrally in a flange of the second coupling member.

11. Apparatus according to any preceding claim, wherein the face of the second wall facing the first wall is tapered.

12. Apparatus according to any preceding claim, wherein the second wall tapers to narrow in thickness towards the mouth of the channel.

13. Apparatus comprising an adhesive pad, an ostomy pouch, and a coupling as defined in any preceding claim, wherein the first coupling member is attached to the pad, and the second coupling member is attached to the pouch.

14. A body-side coupling member for use in the coupling as defined in any of claims 1 to 12.

15. A bag-side coupling member for use in the coupling as defined in any of claims 1 to 12.
